(19) Europäisches Patentamt
European Patent Office
Office européen des brevets

(11)  **EP 2 901 919 B1**

(12)  **EUROPEAN PATENT SPECIFICATION**

(45) Date of publication and mention
of the grant of the patent:
**19.10.2016  Bulletin 2016/42**

(51) Int Cl.:
***A61B 3/10*** *(2006.01)*

(21) Application number: **15153443.5**

(22) Date of filing: **02.02.2015**

(54) **FUNDUS PHOTOGRAPHING APPARATUS AND WIDE-ANGLE LENS ATTACHMENT**

FUNDUSFOTOGRAFIEVORRICHTUNG UND WEITWINKELLINSENBEFESTIGUNG

APPAREIL DE PHOTOGRAPHIE DU FOND DE L' OEIL ET FIXATION DE LENTILLE À GRAND ANGLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.02.2014  JP 2014018717**
**26.12.2014  JP 2014264150**

(43) Date of publication of application:
**05.08.2015  Bulletin 2015/32**

(73) Proprietor: **Nidek Co., Ltd.**
**Aichi 433-0038 (JP)**

(72) Inventor: **Mizuno, Katsuyasu**
**Aichi, 4430038 (JP)**

(74) Representative: **Prüfer & Partner mbB**
**Patentanwälte · Rechtsanwälte**
**Sohnckestraße 12**
**81479 München (DE)**

(56) References cited:
**JP-A- 2009 011 381    US-A1- 2013 057 826**

## Description

Field of the Invention

The present disclosure relates to a fundus photographing apparatus for imaging an image of a fundus, and a wide-angle lens attachment attachable to the fundus photographing apparatus.

Related Art

There is conventionally known a fundus photographing apparatus configured to scan a laser beam on a fundus of an examinee's eye to obtain an image of the fundus. There is known a method used in such an apparatus to photograph an image with a wide photographing angle. In this apparatus, an objective optical system provided between a scanning part and the examinee's eye is operative to bend or deflect the laser beam toward a pivot point. The laser beam pivots or rotates about the pivot point according to operation of the scanning part, thereby scanning the fundus.

For instance, there is proposed a method for widening an imaging view angle of a fundus image by attaching a wide-angle lens attachment near an inspection window of an apparatus main unit. Patent Document 1 discloses an example in which a wide-angle lens attachment having a plurality of convex meniscus lenses is placed with their concave sides facing to an examinee. This wide-angle lens attachment is placed between a single pivot point about which a laser beam having passed through the scanning part is caused to pivot and the inspection window. Accordingly, a laser beam will enter the convex meniscus lenses at a chief ray height lower than when it passes through the inspection window of the apparatus main unit.

Related Art Documents

Patent Documents

Patent Document 1: JP 2009-11381 A

Summary

Problems to be Solved by the Invention

Meanwhile, as the chief ray height of a laser beam entering a lens for widening the angle of a fundus image is lower, a working distance of the apparatus becomes shorter. Thus, in a configuration that a lens attachment is placed between the single pivot point and the inspection window, an interval or distance between the examinee's eye and the lens attachment may be less ensured. This causes problems for example that it is difficult to open an examinee's eye lid in imaging a wide-angle image.

In the apparatus in Patent Document 1, furthermore, even when the wide-angle attachment is attached, the pivot point is single and thus the direction of an image to be created remains unchanged.

When a photographing view angle is to be widened, it is necessary to greatly bend a laser beam by an objective optical system. This optical system has to be designed in consideration of various aberrations which may be caused by such bending.

The present disclosure has been made to solve at least one of the above problems and has a purpose to provide a novel fundus photographing apparatus and a novel wide-angle lens attachment capable of favorably imaging an image of a fundus with a wide view angle.

Means of Solving the Problems

A first aspect of this disclosure provides a fundus photographing apparatus including: a scanning part configured to change a traveling direction of a laser beam emitted from a light source to scan the laser beam on a fundus; and a first objective optical system placed between an examinee's eye and the scanning part to form a first pivot point about which the laser beam delivered from the scanning part is caused to pivot in association with operation of the scanning part, the fundus photographing apparatus being configured to image an image of the fundus of the examinee's eye by use of light from the fundus resulting from the laser beam having passed the first pivot point and being delivered to the fundus of the examinee's eye, characterized in that the apparatus further includes a wide-angle lens attachment attachable to a housing surface of the apparatus on a side which will face to the examinee, the housing surface having an inspection window, and the wide-angle lens attachment being configured to widen an imaging view angle of the image of the fundus, the wide-angle lens attachment is provided with a second objective optical system for forming a second pivot point about which the laser beam having passed the first pivot point is further caused to pivot in association with the operation of the scanning part in an attached state where the attachment is attached to the housing surface, and the second objective optical system includes at least a lens placed to bend the laser beam toward an optical axis of the second objective optical system at a higher position than a chief ray height of the laser beam on the inspection window in the attached state.

A second aspect of this disclosure provides a wide-angle lens attachment including: a scanning part for changing a traveling direction of a laser beam emitted from a light source to scan the laser beam on a fundus of an examinee's eye; and a first objective optical system placed between the examinee's eye and the scanning part and configured to form a first pivot point about which the laser beam delivered from the scanning part is caused to pivot in association with operation of the scanning part, the wide-angle lens attachment being attachable to a housing surface of a fundus photographing apparatus on a side which will face an examinee, the fundus photographing apparatus being configured to image an image of the fundus of the examinee's eye by use of light from the fundus resulting from the laser beam having passed the first pivot point and being delivered to the fundus of the examinee's eye, the attachment being configured to widen an imaging view angle of the image of the fundus, characterized in that a second objective optical system is provided to form a second pivot point about which the laser beam having passed the first pivot point is further caused to pivot in association with the operation of the scanning part in an attached state where the attachment is attached to the housing surface having an inspection window, and the second objective optical system includes at least a lens placed to bend the laser beam toward an optical axis of the second objective optical system at a higher position than a chief ray height of the laser beam on the inspection window in the attached state.

Further developments of the present disclosure are given in the dependent claims.

According to the present disclosure, it is possible to favorably image an image of a fundus with a wide view angle.

BRIEF DESCRIPTION OF THE DRAWINGS

FIG. 1 is a schematic configuration view showing an external appearance of a fundus photographing apparatus in a first embodiment;

FIG. 2 is a schematic diagram showing optical systems in a main unit of the fundus photographing apparatus;

FIG. 3 is a diagram showing a configuration that a wide-angle lens attachment is placed between an examinee's eye and a first objective optical system;

FIG. 4 is a block diagram showing a control system of the fundus photographing apparatus;

FIGs. 5A and 5B are schematic diagrams showing a correspondence relationship between pixels of a fundus image and scanning sequence of a scanning part in an attached state and a non-attached state of a wide-angle lens attachment;

FIG. 6 is a schematic diagram showing a wide-angle lens attachment in a second embodiment;

FIG. 7 is a schematic diagram showing a wide-angle lens attachment in a third embodiment;

FIGs. 8A and 8B are diagrams showing modified examples of the wide-angle lens attachment in the third embodiment, FIG. 8A illustrating an example of a lens surface formed as a concave surface and FIG. 8B illustrating an example of a lens surface formed as a convex surface;

FIGs. 9A and 9B are diagrams showing differences on design of the wide-angle lens attachment according to orientation of a second objective lens in the third embodiment; and

FIG. 10 is a schematic diagram showing an optical system in a photographing apparatus configured to change a photographing view angle by replacement of objective lenses, to which the objective lens optical system of the present disclosure is applied.

DETAILED DESCRIPTION OF THE EXEMPLARY EMBODIMENTS

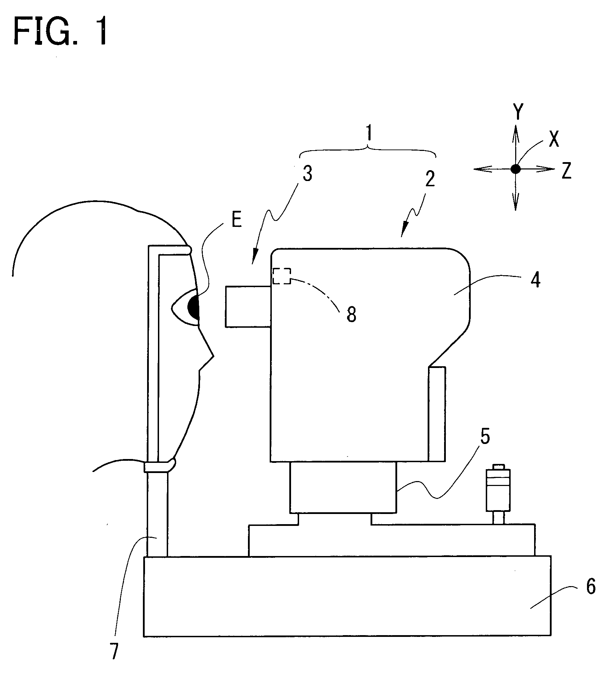

Typical embodiments of this disclosure will be explained below referring to accompanying drawings. A schematic configuration of a fundus photographing apparatus 1 in a first embodiment will be first explained referring to FIG. 1. The first embodiment explained below uses a Scanning Laser Ophthalmoscope (SLO) as the fundus photographing apparatus 1.

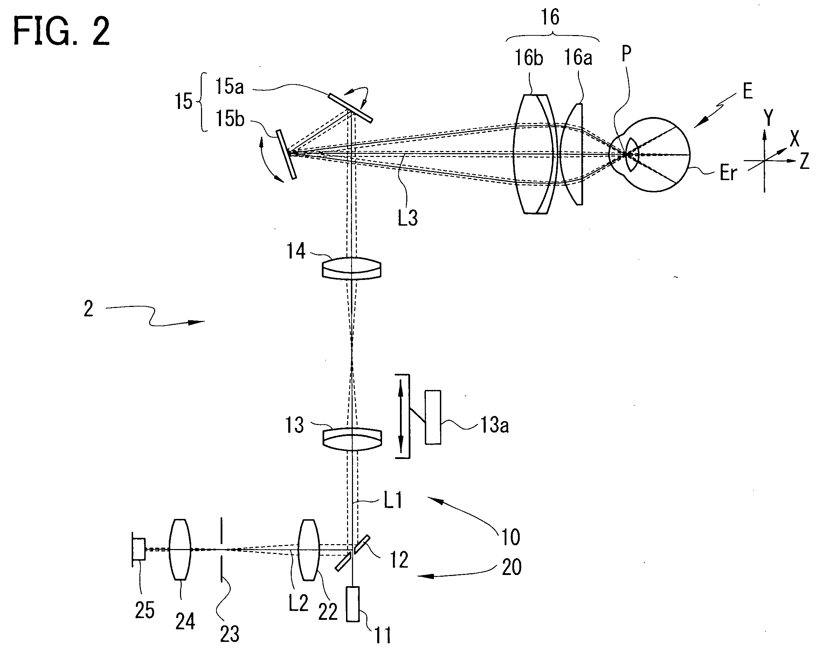

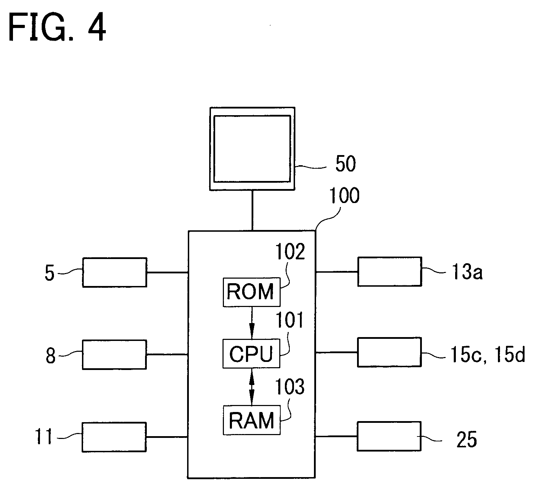

As shown in FIG. 1, the fundus photographing apparatus 1 includes a main unit (an apparatus main unit) 2 and a wide-angle lens attachment 3. In the first embodiment, the main unit 2 has optical systems (see FIG. 2) and a control system (see FIG. 4) which are essential in imaging a fundus image. In the first embodiment, further, the wide-angle lens attachment 3 is configured to be attachable to a housing surface of the main unit 2 which will face to an examinee. When attached to the main unit 2, the wide-angle lens attachment 3 is operative to widen an imaging view angle of a fundus image to be obtained by the main unit 2.

In the first embodiment, the main unit 2 includes a measuring part 4, an alignment mechanism 5, a base table 6, a face support unit 7, and a sensor 8. The measuring part 4 accommodates therein the optical systems for imaging an examinee's eye E. The optical systems will be described later with reference to FIG. 2.

The alignment mechanism 5 is used to align the apparatus with the examinee's eye E. The alignment mechanism 5 in the present embodiment is configured to move the measuring part 4 in three dimensions with respect to the base table 6, that is, in each of a Y direction (up and down direction), an X direction (right and left direction), and a Z direction (back and forth direction).

The face support unit 7 is used to support the face of an examinee to direct the examinee's eye E at the measuring part 4 as shown in FIG. 1. In the first embodiment, the face support unit 7 is provided on the base table 6.

The sensor 8 is detecting means for detecting attachment of the wide-angle lens attachment 3. The sensor 8 outputs an electric signal representing an attached state of the wide-angle lens attachment 3. For instance, the sensor 8 continuously outputs electric signals different in voltage value between when the wide-angle lens attachment 3 is attached to the main unit 2 and when the wide-angle lens attachment 3 is not attached to the main unit 2. Such a sensor 8 may be a contact sensor such as a micro switch and further for example may be a non-contact sensor such as a photoelectric sensor and a magnetic sensor.

The fundus photographing apparatus 1 may also be an apparatus integrally combined with another ophthalmic apparatus such as an Optical Coherence Tomography (OCT) and a perimeter.

The optical systems in the main unit 2 will be explained referring to FIG. 2. This figure shows the main unit 2 not attached with the wide-angle lens attachment 3.

In the first embodiment, the main unit 2 includes a light projecting optical system 10 and a light receiving optical system 20. The light projecting optical system 10 is configured to project a laser beam (measurement light) to each point in a range on a fundus Er of the examinee's eye E to be imaged. In the first embodiment, the light projecting optical system 10 includes a laser beam emitting part 11, a perforated mirror 12, a lens 13, a lens 14, a scanning part 15, and a first objective optical system 16.

The laser beam emitting part 11 is a light source (i.e., a laser light source) of the light projecting optical system 10. In the first embodiment, the laser beam emitting part 11 will be explained as emitting only single-color light for convenience of explanation.

The laser beam from the laser beam emitting part 11 passes through an aperture of the perforated mirror 12 and then the lenses 13 and 14, and travels toward the scanning part 15. The light beam reflected by the scanning part 15 passes through the first objective optical system 16 and then converges on the fundus Er of the examinee's eye E. Consequently, the light scattered and reflected by the fundus Er (hereinafter, referred to as fundus reflection light) emerges from a pupil.

In the first embodiment, the lens 13 is arranged to be movable in a direction of an optical axis L1 by a drive mechanism 13a (see FIG. 4). According to the position of the lens 13, the diopters of the light projecting optical system 10 and the light receiving optical system 20 are changed. In the first embodiment, therefore, an error in diopter of the examinee's eye E is corrected (reduced) by adjustment of the position of the lens 13. Of course, the lens 14 may be displaced to correct the diopter error of the examinee's eye E.

The scanning part 15 is a unit for changing a traveling direction of the laser beam (bending the laser beam) delivered from the laser beam emitting part 11 in order to scan the laser beam on the fundus. In the first embodiment, the scanning part 15 includes a resonant scanner 15a and a galvano mirror 15b.

As the scanning part 15, for example, there may be used an acousto-optical modulator (AOM) or the like for changing the traveling (bending) direction of light as well as a reflection mirror (a galvano mirror, a polygon mirror, and a resonant scanner).

In the first embodiment, the resonant scanner 15a bends the laser beam to be projected onto the fundus of the examinee's eye E in a predetermined direction. As shown in FIG. 2, the light having passed through the resonant scanner 15a travels toward the galvano mirror 15b.

In the first embodiment, when the resonant scanner 15a is rotated by a motor 15c (see FIG. 4), an irradiation position (a scanning position) of the laser beam on the fundus Er is moved in a horizontal direction (i.e., the X direction). In the first embodiment, main scanning in the X direction is performed by the resonant scanner 15a.

In the first embodiment, furthermore, the galvano mirror 15b further bends the laser beam having passed the resonant scanner 15a in a direction different from that by the resonant scanner 15a. As shown in FIG. 2, the light having passed the galvano mirror 15b travels toward the first objective optical system 16. In the first embodiment, the galvano mirror 15b is rotated by a motor 15d (see FIG. 4), thereby moving the irradiation position of the laser beam on the fundus Er in a vertical direction (i.e., the Y direction). In the first embodiment, sub-scanning in the Y direction is performed by the galvano mirror 15b. In the first embodiment, the main scanning in the X direction by the resonant scanner 15a and the sub-scanning in the Y direction by the galvano mirror 15b are performed in a predetermined scanning order to scan the laser beam on the fundus Er in two dimensions.

The first objective optical system 16 is configured to make the laser beam having passed the scanning part 15 pass through a pupil position of the examinee's eye E in a state of the main unit 2 not attached with the wide-angle lens attachment 3. The first objective optical system 16 has positive power. For example, the first objective optical system 16 in the first embodiment includes two convex lenses (a first convex lens 16a and a second convex lens 16b) arranged in series. The number of lenses included in the first objective optical system 16 is not limited to that of the above configuration. For instance, the first objective optical system 16 may be configured to have a single lens or have three or more lenses. Each lens of the first objective optical system 16 may also be an aspherical lens, a compound lens consisting of a plurality of lenses, or the like, as needed for aberration correction. The first objective optical system 16 may also include an optical member (e.g., a reflection mirror) other than a lens.

In the first embodiment, the first objective optical system 16 forms a first pivot point P on an optical axis L3 of the first objective optical system 16 so that the laser beam (more concretely, the chief ray of the laser beam) having passed the scanning part 15 is caused to pivot about the first pivot point P. In the first embodiment, the first pivot point P is formed at an optically conjugated position with the scanning part 15 (e.g., a middle point between the resonant scanner 15a and the galvano mirror 15b) through the first objective optical system 16. The laser beam having passed the scanning part 15 then passes through the first objective optical system 16, and is irradiated to the fundus Er via the first pivot point P. Accordingly, the chief ray of the laser beam having passed through the first objective optical system 16 is caused to pivot about the first pivot point P in association with the operation of the scanning part 15. In the example shown in FIG. 2, consequently, the laser beam is scanned two-dimensionally on the fundus Er. Since the first pivot point P of the laser beam and the pupil position of the examinee's eye E are made to coincide with each other in advance, vignetting by the iris is reduced and the laser beam is reliably delivered to the fundus reducing. This enables imaging a good fundus image.

The light receiving optical system 20 will be explained below. The light receiving optical system 20 is configured so that the fundus reflection light emerging from the pupil in association with the laser beam from the light projecting optical system 10 is received by a light receiving element 25. The light receiving optical system 20 in the first embodiment shares each component placed on an optical path of the light projecting optical system 10 from the perforated mirror 12 to the first objective optical system 16, with the light projecting optical system 10. The light receiving optical system 20 in the first embodiment further includes a lens 22, a pinhole plate 23, a lens 24, and the light receiving element 25.

When the laser beam is to be irradiated to the fundus of the examinee's eye E, the fundus reflection light of the laser beam travels back through the above-mentioned light projecting optical system 10 and is reflected by the perforated mirror 12 toward the lens 22. The pupil position of the examinee's eye E and the aperture of the perforated mirror 12 are in an optically conjugated relationship. On a downstream side of the lens 22, the light from the fundus Er focuses on a pinhole of the pinhole plate 23 and then is received by the light receiving element 25 through the lens 24. The first embodiment uses, as the light receiving element 25, an APD (avalanche photodiode) sensitive to a visible region and an infrared region.

The optical systems in the main unit 2 in the first embodiment are configured as above. In the first embodiment, a fundus image of one frame is obtained based on scanning of the laser beam on the fundus Er corresponding to one frame.

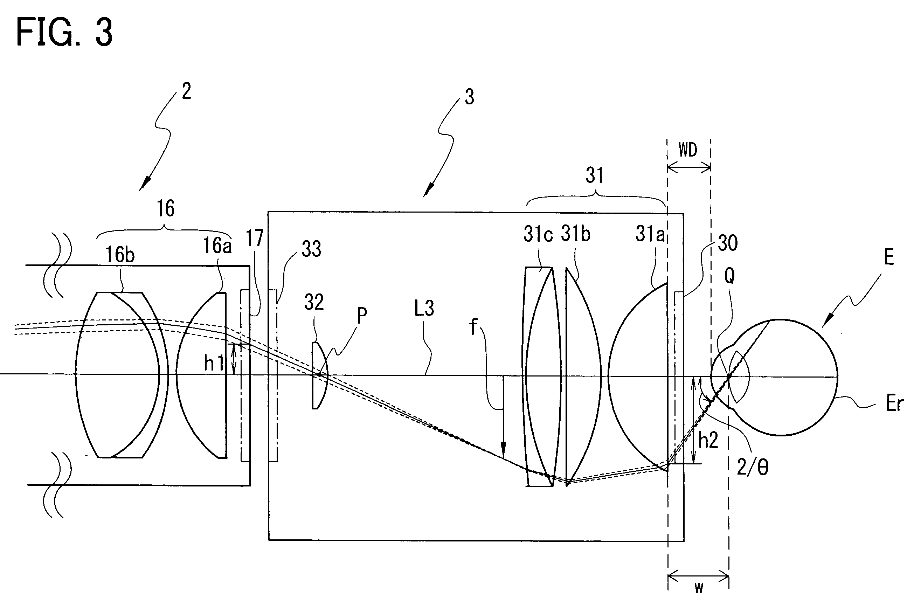

Next, the optical systems in the main unit 2 attached with the wide-angle lens attachment 3 will be explained referring to FIG. 3. In FIG. 3, 17 denotes an inspection window of the main unit 2. Further, 30 denotes an inspection window of the wide-angle lens attachment 3, and 33 denotes an entrance window of the wide-angle lens attachment 3. The inspection windows and the entrance window are illustrated in positions away from the lens surfaces for convenience; however, the lens surfaces may also be used as the inspection window and others. For instance, the lens 16a may also be used as the inspection window 17 of the main unit 2. Further, a lens 31a may also be used as the inspection window of the wide-angle lens attachment 3. In the first embodiment, each of the inspection windows and the entrance window may be fitted with a cover glass for dust prevention.

In the first embodiment, the wide-angle lens attachment 3 is attached to a housing surface of the main unit 2 on a side facing to the examinee so that a second objective optical system 31 which will be mentioned later is placed in the optical path of the laser beam. For instance, the wide-angle lens attachment 3 is disposed near the inspection window 17 located between the first objective optical system 16 and the first pivot point P.

As shown in FIG. 3, the wide-angle lens attachment 3 mainly includes the second objective optical system 31. The wide-angle lens attachment 3 in the first embodiment further includes a diopter correcting lens 32 (a diopter correcting lens optical system). In an attached state in which the wide-angle lens attachment 3 is attached to the main unit 2, the above-mentioned optical systems included in the wide-angle lens attachment 3 are placed between the inspection window 17 of the main unit 2 and the examinee's eye E.

In the first embodiment, in the attached state of the wide-angle lens attachment 3, the second objective optical system 31 and the diopter correcting lens 32 are placed on the optical path of the laser beam. In the attached state of the wide-angle lens attachment 3, the laser beam passing through the first objective optical system 16 of the main unit 2 and then entering the wide-angle lens attachment 3 through the inspection window 17 and the entrance window 33 travels through the diopter correcting lens 32 and the first pivot point P, and then passes through the second objective optical system 31. In the first embodiment, the diopter correcting lens 32 is placed at the first pivot point P formed by the first objective optical system 16, the details of which will be mentioned later. This allows the laser beam to pass through near the center of the diopter correcting lens 32. Thus, the inclination and the height of the laser beam are less influenced by the power of the diopter correcting lens 32.

The second objective optical system 31 forms a second pivot point Q while the wide-angle lens attachment 3 is in the attached state. The second pivot point Q is a point about which the laser beam (more concretely, the chief ray of the laser beam) having passed the first pivot point P is further caused to pivot in association with the operation of the scanning part 15. In the first embodiment, the second pivot point Q is formed at an optically conjugated position with the scanning part 15 (e.g., the middle point between the resonant scanner 15a and the galvano mirror 15b) through the second objective optical system 31. The second pivot point Q is formed on the optical axis of the second objective optical system 31. In the first embodiment, the optical axis of the second objective optical system 31 is coaxial with the optical axis L3 and therefore a reference sign L3 is used to indicate the optical axis of the second objective optical system 31.

In the first embodiment, the second objective optical system 31 includes, in the order of being placed near the examinee's eye, a first objective lens 31a, a second objective lens 31b, and a third objective lens 31c. The second objective optical system 31 in the first embodiment may use a lens optical system having a larger effective aperture with respect to a maximum value (h1max) of the chief ray height h1 of the laser beam which passes through the inspection window 17. For example, the first objective lens 31a and the second objective lens 31b are lenses each having a larger effective aperture than the first convex lens 16a. The third objective lens 31c may also be a lens having a larger effective aperture than the first convex lens 16a. In the first embodiment, however, h1 represents the chief ray height with respect to the optical axis L3 of the first objective optical system 16.

The second objective optical system 31 (each lens 31a to 31c) is placed on a side closer to the examinee's eye E than the first pivot point P as shown in FIG. 3. To be concrete, the second objective optical system 31 in the first embodiment is placed so that an interval or distance between a nearest lens surface to a light source (the lens surface of the third objective lens 31c on a side close to a light source in the first embodiment) and the first pivot point P is larger than an interval or distance between the inspection window 17 and the first pivot point P. This allows the laser beam having a high chief ray height with respect to the laser beam passing through the inspection window 17 to enter the lens surface placed on the nearest side to the light source in the second objective optical system 31.

In the first embodiment, each of the objective lenses 31a to 31c has positive power. However, not limited thereto, the second objective optical system 31 has only to have positive power in total. The second objective optical system 31 includes at least a lens (e.g., each objective lens 31a, 31b, 31c in the first embodiment) placed to bend the laser beam toward the optical axis L3 of the second objective optical system 31 at a higher position than the chief ray height h1 of the laser beam in the inspection window 17. This lens serves to bend the laser beam having passed the first pivot point P. The lens placed to bend the laser beam bends the chief ray traveling in a direction away from the optical axis L3 to travel in a direction towards the optical axis L3. In the first embodiment, the laser beam bent by the third objective lens 31c and the second objective lens 31b is caused to enter the first objective lens 31a and further is bent at a sharp angle with respect to the optical axis L3. Accordingly, the pivot angle of the laser beam caused to pivot about the second pivot point Q is larger than that of the laser beam caused to pivot about the first pivot point P. Thus, the fundus photographing apparatus 1 enables providing a wider imaging view angle in the attached state of the wide-angle lens attachment 3 than in a non-attached state. For instance, when the imaging view angle in the attached state of the wide-angle lens attachment 3 is about 30° to 60°, the imaging view angle in the attached state can be widened to about 90° to about 150° in full view angle.

Herein, as shown in FIG. 3, assuming that the chief ray height of the laser beam passing through the lens surface on the nearest side to the examinee's eye E (the lens surface of the first objective lens 31a facing to the examinee in the first embodiment) is h2 (where h2 denotes a chief ray height with respect to the optical axis L3 of the second objective optical system 31), a working distance WD in the fundus photographing apparatus 1 is expressed by the following expression (1):

$$WD = w - \beta$$

where

$$WD = h2max / \tan(\theta/2) \quad ....(1)$$

The working distance WD defined herein is a distance from a corneal vertex to a lens surface closest to the examinee's eye. w represents a distance from a pivot point set in a pupil to a lens surface closest to the examinee's eye. An approximate working distance WD is obtained by subtracting $\beta$ (e.g., 3 mm) which is a typical distance from a corneal vertex to a pupil of a human eye from w. Further, h2max is a maximum value of the chief ray height h2 of the laser beam on the lens surface closest to the examinee's eye. $\theta$ is a pivot angle (an angle of pivot range). As expressed by the expression (1), the working distance WD is longer as h2max is larger.

Herein, a conventional wide-angle lens attachment (see Patent Document 1) is configured such that a lens for widening an imaging view angle is placed in a position where a chief ray height of a laser beam is lower than when the laser beam passes through an inspection window of an apparatus main unit. In the conventional apparatus, therefore, the chief ray height of the laser beam (laser beam) on a lens surface closest to an examinee's eye is always lower than a chief ray height of a laser beam on an inspection window of the main unit. In contrast, in the fundus photographing apparatus 1 in the first embodiment, the lenses 31a to 31c are each placed in the positions to make higher the chief ray height of the laser beam than the chief ray height h1 on the inspection window 17. In the first embodiment, accordingly, the chief ray height of the laser beam on the lens surface closest to the examinee's eye is more easily ensured as compared with the conventional apparatus. For example, it may also be designed such that the chief ray height h2 of the laser beam on the lens surface closest to the examinee's eye E is higher than the chief ray height h1 of the laser beam on the inspection window 17, as shown in FIG. 3. Accordingly, the fundus photographing apparatus 1 in the first embodiment enables ensuring the interval or distance between the examinee's eye and the wide-angle lens attachment 3 while the wide-angle lens attachment 3 is attached to the main unit 2.

Meanwhile, for making wider a photographing view angle of a fundus image, it is also conceivable that an objective lens of the apparatus main unit is replaced with a lens for wide-angle photographing. However, this manner needs detachment of the objective lens once from the main unit in order to change the photographing view angle of the fundus image. Further, during detachment of the objective lens, the inside of the main unit is exposed. In contrast, the fundus photographing apparatus 1 in the first embodiment can switch the photographing view angle of the fundus image by attaching or detaching the wide-angle lens attachment 3 without detaching the objective lens from the main unit. This fundus photographing apparatus 1 in the first embodiment allows an examiner or another person to switch the photographing view angle in a simple way. The fundus photographing apparatus 1 in the first embodiment further enables dust-prevention property of the main unit even when the wide-angle lens attachment 3 is attached or detached.

The second objective optical system 31 is not limited to the above configuration and may be configured to include an aspherical lens, a compound lens consisting of a plurality of lenses, and others. Furthermore, the second objective optical system 31 may also be configured to have a single lens (e.g., an aspherical lens) or have two lenses or four or more lenses.

The diopter correcting lens 32 cancels out diopter change caused by the second objective optical system 31 (that is, cancels out part or all diopter changes). To be concrete, the diopter correcting lens 32 suppresses a difference in diopter of the light entering the examinee's eye E between the attached state and the non-attached state of the wide-angle lens attachment 3. In the first embodiment, a lens having positive power (concretely, a plano-convex lens with a convex surface oriented toward the examinee's eye E) is used as the diopter correcting lens 32.

Herein, for example, there is a conceivable case where diopter change by the second objective optical system 31 is large (e.g., about several tens diopters) with respect to an accommodation amplitude of the diopter in the optical systems of the main unit 2 (e.g., an accommodation amplitude of the diopter by movement of the lens 13). In the first embodiment, accordingly, the diopter correcting lens 32 cancels out the diopter change to be caused by the second objective optical system 31. Thus, even when the diopter change caused by the second objective optical system 31 is larger than the diopter accommodation amplitude in the optical systems of the main unit 2, a fundus image with wide angle can be imaged well. Since the diopter correcting lens 32 reduces the difference in diopter of the light entering the examinee's eye E between the attached state and the non-attached state of the wide-angle lens attachment 3, diopter accommodation of the apparatus in association with attachment/detachment of the wide-angle lens attachment 3 with respect to the main unit 2 is simplified or is rendered unnecessary.

Moreover, as shown in FIG. 3, in the first embodiment, the diopter correcting lens 32 is placed on a side closer to the light source relative to the second objective optical system 31. To be concrete, the diopter correcting lens 32 in the first embodiment is placed at the first pivot point P. This allows the laser beam to pass through near the center of the diopter correcting lens 32. Thus, a small-diameter lens can be used as the diopter correcting lens 32. In the first embodiment, furthermore, the diopter correcting lens 32 has positive power. However, the laser beam passes through near the center of the diopter correcting lens 32. Accordingly, the inclination and the height of the laser beam are less influenced by the power of the diopter correcting lens 32. This prevents an increase in the distance between the inspection window 17 and the position in which the chief ray height of the laser beam having passed the first pivot point P becomes higher than h1. Specifically, the distance between the second objective optical system 31 and the inspection window 17 is reduced, thereby avoiding an increase in the length of the wide-angle lens attachment 3. The first embodiment is explained with the diopter correcting lens 32 located at the first pivot point P, but it is not limited thereto. As an alternative, the diopter correcting lens 32 may be placed in another position inside the wide-angle lens attachment 3. However, it is preferable that the diopter correcting lens 32 is not placed in a converging point on which the laser beam converges (e.g., a position in which the intermediate image f is formed in FIG. 3) within the attachment.

In the first embodiment, the state where the diopter correcting lens 32 is located at the first pivot point P includes not only a case where the position of the diopter correcting lens 32 exactly coincides with the position of the first pivot point P but also a case where the diopter correcting lens 32 is somewhat displaced forward or backward with respect to the first pivot point P. An allowable amount of displacement can be appropriately set in view of a relationship with the required accuracy.

Next, referring to FIG. 4, a control system of the fundus photographing apparatus 1 will be explained. Main control of the fundus photographing apparatus 1 is performed by a control unit 100. This control unit 100 is a processing unit having an electronic circuit to execute a control processing of each part of the fundus photographing apparatus 1 and a calculation processing of measurement results.

In the first embodiment, the control unit 100 is connected to the alignment mechanism 5, the sensor 8, the motors 15c and 15d, the light receiving element 25, and the monitor 50.

The control unit 100 includes a CPU 101, a ROM 102, and a RAM 103. The CPU 101 is a processing device to execute various processings related to the fundus photographing apparatus 1. The ROM 102 is a nonvolatile storage device which stores various control programs and fixed data. The ROM 103 is a rewritable volatile storage device. This ROM 103 stores for example temporary data to be used in photographing and measuring the examinee's eye E.

Meanwhile, according to the wide-angle lens attachment 3 in the first embodiment, the laser beam is bent by the second objective optical system 31, thereby making the orientation of the laser beam before the second objective optical system 31 to reverse right to left (horizontally) and top to bottom (vertically) after passing through the second objective optical system 31. That is, the inclination of the laser beam at the first pivot point P and the inclination of the laser beam at the second pivot point Q are reversed with respect to the optical axis L3. Therefore, for example, when the same photographing method and the same image creating method are adopted in both the attached state and the non-attached state of the wide-angle lens attachment 3, in one of the attached state and the non-attached state of the wide-angle lens attachment 3, an erected image is created in the same orientation in vertical and lateral directions as the orientation of the fundus, while in the other state, an inverted image is created in the reversed orientation in the vertical and lateral directions from the orientation of the fundus. In the fundus photographing apparatus 1 in the first embodiment, for example, the erected image is obtained in the non-attached state of the wide-angle lens attachment 3, while the inverted image is obtained in the attached state of the wide-angle lens attachment 3.

Accordingly, the control unit 100 in the first embodiment performs the image processing to create a fundus image by vertically and horizontally reversing pixel arrangement corresponding to the scanning order of the scanning part 15 from the fundus image imaged in the non-attached state of the wide-angle lens attachment 3. This image processing in the first embodiment is performed based on input of a detection signal representing the attached state of the wide-angle lens attachment 3 input from the sensor 8 to the control unit 100. Specifically, the inverted image imaged in the attached state of the wide-angle lens attachment 3 is subjected to the processing.

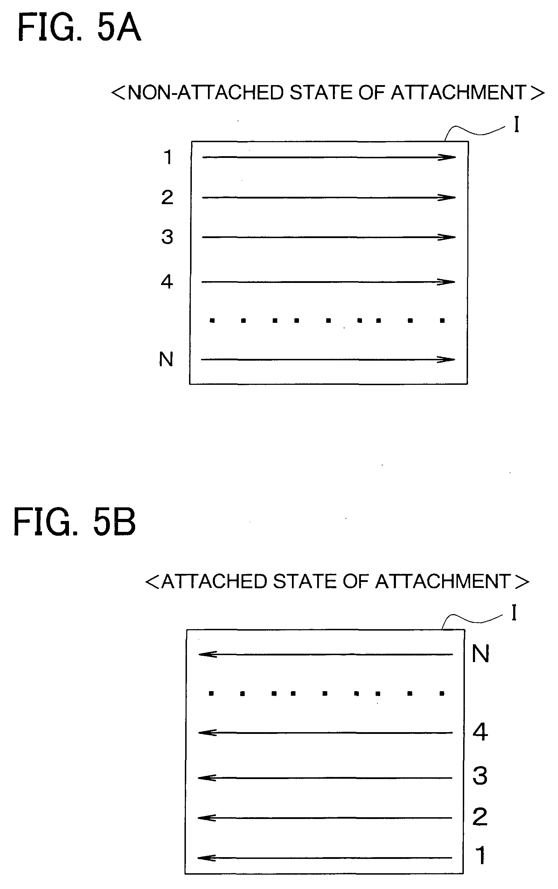

Herein, the corresponding relationship between pixels of a fundus image I and the scanning order of the scanning part 15 in the attached state and the non-attached state of the wide-angle lens attachment 3 is shown as one example in FIGs. 5A and 5B. In FIGs. 5A and 5B, arrows on the image indicate the order of main scanning by the scanning part 15 and numerals assigned to the arrows represent the order of sub-scanning by the scanning part 15. In the fundus image obtained in the non-attached state of the wide-angle lens attachment 3 (see FIG. 5A), the pixels formed based on the received light signal at each timing in the light receiving element 25 are arranged in time sequence (i.e., in association with the order of main scanning) from left to right in one row at a time. Further, the rows are arranged from top to bottom in time sequence (i.e., in association with the order of sub-scanning). In the attached state of the wide-angle lens attachment 3, on the other hand, the above-mentioned reversal processing by the control unit 100 is performed so that the pixels formed based on the received light signal at each timing in the light receiving element 25 are arranged in time sequence (i.e., in association with the order of main scanning) from right to left in one row at a time. Further, the rows are arranged from bottom to top in time sequence (i.e., corresponding to the order of sub-scanning). By this processing, the corresponding relationship between the vertical and lateral orientation of the image and the vertical and lateral orientation of the fundus is made coincident between the attached state and the non-attached state of the wide-angle lens attachment 3.

In the first embodiment, the inverted fundus image obtained in the attached state of the wide-angle lens attachment is subjected to the image processing of vertically and horizontally reversing the orientation of the image. In the first embodiment, the image processing is executed according to a detection result of the sensor 8. The sensor 8, as described above, serves to detect the presence/absence of attachment of the wide-angle lens attachment 3 with respect to the main unit 2. For instance, when the control unit 100 receives a signal output from the sensor 8 when the wide-angle lens attachment 3 is attached, the image processing of vertically and horizontally reversing the image is performed by the control unit 100. In the first embodiment, as a result of the image processing, the fundus image imaged with a wide angle is also obtained as an erected image. Thus, the erected image of the fundus image can be acquired irrespective of the presence/absence of attachment of the wide-angle lens attachment 3.

The first embodiment is explained as the case where the above-mentioned image processing is performed as the reversal processing, but it is not limited thereto. For example, instead of vertically and horizontally reversing the orientation of the fundus image created once, it may be arranged to create a fundus image having been reversed vertically and horizontally in advance. The reversal processing may be reversal processing by software or reversal processing by hardware (e.g., a reversal processing circuit using an electric circuit).

The control unit 100 also performs display control to display a fundus image sequentially created as a live image on the monitor 50. In the first embodiment, for example, when the wide-angle lens attachment 3 is attached to the main unit 2, the control unit 100 causes the monitor 50 to display, as a live image, a fundus image in a reversed orientation by the above-mentioned image processing. On the other hand, when the wide-angle lens attachment 3 is not attached, the control unit 100 causes the monitor 50 to display, as a live image, a fundus image not subjected to the reversal processing. The reversal processing may also be carried out to display not only the live image but also a static image of the fundus created by the main unit 2.

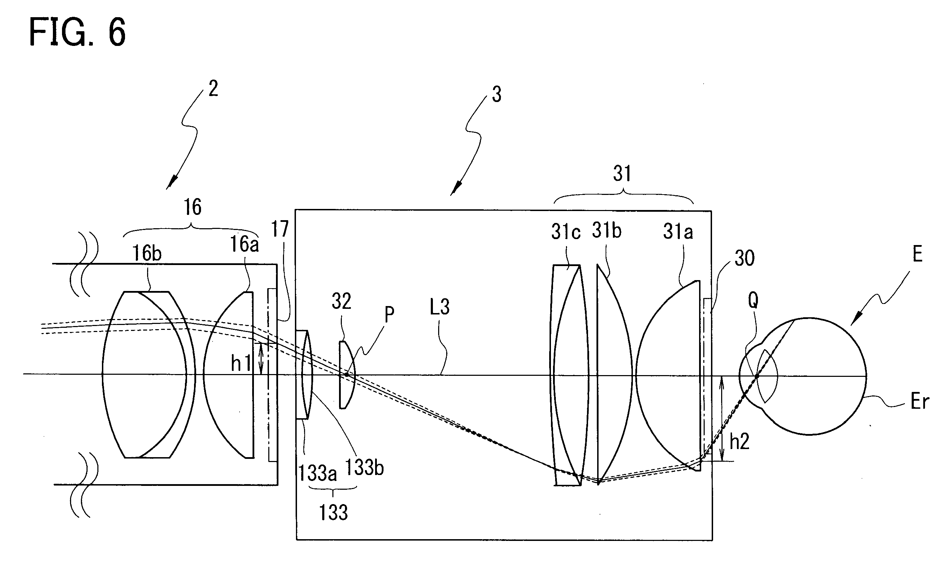

A second embodiment of the present disclosure will be explained below referring to FIG. 6. In the second embodiment, similar or identical parts to those in the first embodiment are explained with the same reference signs as those in the first embodiment, and their explanations are omitted. In the second embodiment, the fundus photographing apparatus 1 is attached with a wide-angle lens attachment 3 having a different lens configuration from that in the first embodiment. Accordingly, the fundus photographing apparatus 1 in the second embodiment includes a more preferable configuration in photographing a fundus image by use of laser beams in multiple colors. In the second embodiment, for instance, the fundus photographing apparatus 1 may be configured to make the laser beam emitting part 11 to output a light beam created by synthesizing monochromatic lights of three colors, red, green, and blue to image a color image of a fundus. In this case, the light receiving optical system 20 of the fundus photographing apparatus 1 may be provided with separate light receiving elements sensitive to different single colors and also with an appropriate configuration (e.g., a dichroic mirror) for dividing an optical path so as to allow the corresponding light receiving elements to receive respective wavelength lights.

In the configuration to create an image based on the lights of a plurality of wavelengths as mentioned above, lateral (magnification) chromatic aberration of lenses included in the optical system of the apparatus will become problematic. Thus, the wide-angle lens attachment 3 shown in FIG. 6 is provided with a lateral chromatic aberration (LCA) correcting lens 133. This LCA correcting lens 133 serves to reduce at least lateral (magnification) chromatic aberration of a laser beam generated by the second objective optical system 31. The LCA correcting lens 133 may be an achromatic lens formed by bonding two or more lenses different in refraction and light dispersion (Abbe's number). For instance, in the example in FIG. 6, a concave lens 133a made of flint glass and a convex lens 133b made of crown glass are bonded to make the LCA correcting lens 133. The number of lenses to make the LCA correcting lens 133 may be determined according to the number of colors included in the laser beam. For instance, the LCA correcting lens 133 in FIG. 6 can align focal positons of two out of three color lights included in the laser beam to a focal position of remaining one color light. In the example in FIG. 6, accordingly, it is possible to reduce the influence of the lateral chromatic aberration to the fundus image obtained by the apparatus.

As the LCA correcting lens 133, a lens with power zero (that is, the sum of powers of the concave lens 133a and the convex lens 133b is zero) is preferably used. In this case, the LCA correcting lens 133 is less likely to have an influence on the inclination and the height of the light beam and others. Thus, the presence/absence of the LCA correcting lens 133 less influences the design of the second objective optical system 31.

In the example of FIG. 6, the LCA correcting lens 133 is placed between the inspection window 17 of the main unit 2 and the first pivot point P in the attached state of the wide-angle lens attachment 3 and also constitutes an entrance window for laser beam in the wide-angle lens attachment 3. This configuration can protect the wide-angle lens attachment 3 from dust even when no cover glass or the like is provided as the entrance window of the wide-angle lens attachment 3. Of course, another configuration that the LCA correcting lens 133 is placed away from the entrance window may also be adopted.

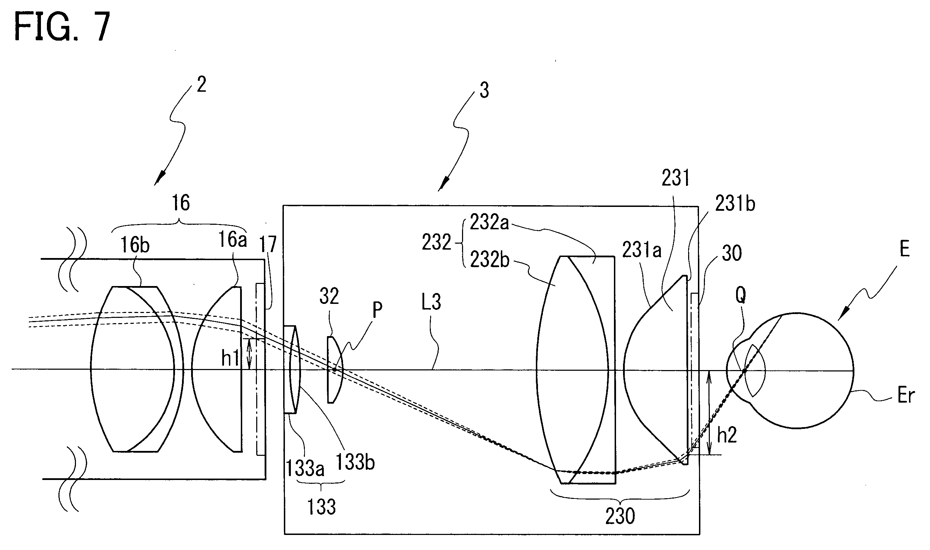

A third embodiment of the present disclosure will be explained below referring to FIG. 7. A fundus photographing apparatus 1 in the third embodiment differs in the lens configuration of the second objective optical system 230 provided in the wide-angle lens attachment 3 from the second objective optical system 31 in the first and second embodiments. In the third embodiment, similar or identical parts to those in the second embodiment are explained with the same reference signs as those in the second embodiment and their explanations are omitted.

The wide-angle lens attachment 3 in the third embodiment is provided with a second objective optical system 230. Similar to the second embodiment, furthermore, the wide-angle lens attachment 3 in the third embodiment may be provided with the diopter correcting lens 32 and the LCA correcting lens 133 as shown in FIG. 7.

The second objective optical system 230 in the third embodiment includes a first objective lens 231 and a second objective lens 232 in the order of being placed close to an examinee's eye (see FIG. 7). In the third embodiment, the first objective lens 231 and the second objective lens 232 are arranged adjacently. In an example of FIG. 7, both the first objective lens 231 and the second objective lens 232 are lenses having positive power. In the attached state of the wide-angle lens attachment 3 in the third embodiment, each of the lenses 231 and 232 is utilized to bend a laser beam toward the optical axis L3 of the second objective optical system 230 at a higher position than the chief ray height of the laser beam on the inspection window 30.

In the third embodiment, the first objective lens 231 is an aspherical lens. This first objective lens 231 corrects spherical aberration of imaging at the second pivot point Q (that is, a pivot point formed by the second objective optical system 230).

In a scanning laser ophthalmoscope, commonly, to rotate the laser beam at an anterior segment and then deliver the laser beam to a fundus, an objective optical system (e.g., the objective optical systems 16, 31, 230, etc.) for bending the laser beam needs to be provided between the scanning part and the examinee's eye as in the present disclosure. In this case, when the laser beam is caused to pass through and pivot about one point (the pivot point) of the anterior segment (e.g., a pupil) with accuracy, an examinee's eye with a non-mydriatic fundus (that is, in a state not instilled with mydriatic drops) can be subjected to fundus photographing with wide view angle. Herein, it is essential to reduce spherical aberration of imaging at the pivot point in order to guide the laser beam bent by the objective optical system into an eye. Specifically, since the spherical aberration is reduced, the laser beam is less likely to be vignetted by the pupil. This spherical aberration is more apt to occur when a light beam is bent at a sharp angle by the objective optical system, that is, when a photographable view angle is large. For instance, in a case of ensuring a view angle of 90° or more in full angle, when the spherical aberration of imaging at the pivot point is to be reduced by use of only a spherical lens(es), a plurality of spherical lenses are required, resulting in a complicated apparatus configuration. Further, if the spherical aberration of imaging at the pivot point is large, the laser beam is likely to be vignetted by the pupil of the examinee's eye. This results in a decreased alignment permissible range in which a fundus image with uniform brightness is obtainable (that is, a permissible range of positional adjustment between the examinee's eye and the apparatus).

In the third embodiment, in contrast, the spherical aberration of imaging at the second pivot point Q (that is, the pivot point formed by the second objective optical system 230) is reduced by the first objective lens 231 which is the aspherical lens. Consequently, for example, a smaller number of lenses can constitute the objective optical system 230 for reliably reducing the spherical aberration. Since the laser beam is less likely to be vignetted by the pupil in association with reduction in spherical aberration, the alignment permissible range is widened. Thus, a fundus image with uniform brightness is easily obtained.

As shown in FIG. 7, the first objective lens 231 may also be configured such that at least a lens surface 231a located on a side close to the light source is aspherical. In this case, the lens surface 231a is formed with a curved surface having a radius curvature increasing as being away from the optical axis L3 of the first objective lens 231 to reduce the spherical aberration.

On the other hand, the first objective lens 231 may have a planar lens surface 231b located on a side close to an examinee's eye. As the result of study made by the present inventor, it is confirmed that as the examinee's-eye-side lens surface 231b has a larger curvature radius, the distance or interval w between the lens surface 231b (especially, a position of a closest portion of the lens surface 231b to the examinee's eye) and the pivot point Q could be ensured more easily. In the example of FIG. 7, the examinee's-eye-side lens surface 231 b is planar and has an infinite curvature radius. This enables easy design of an optical system whereby a sufficient distance w is obtained. As a result that the examinee's-eye-side lens surface 231 b is formed as a surface having a sufficiently large curvature radius, the working distance WD is easily ensured. Accordingly, for instance, it is easy for an examiner or an assistant or the like to open an eyelid of the examinee's eye (to lift up an upper eyelid with fingers or the like). One design solution of the optical system in FIG. 7 by the inventor enables ensuring a distance w of about 13 mm while providing a view angle of 100° or more in full angle. Herein, in a case where the lens surface 231b is also used as the inspection window 30, a working distance WD of about 10 mm can be ensured.

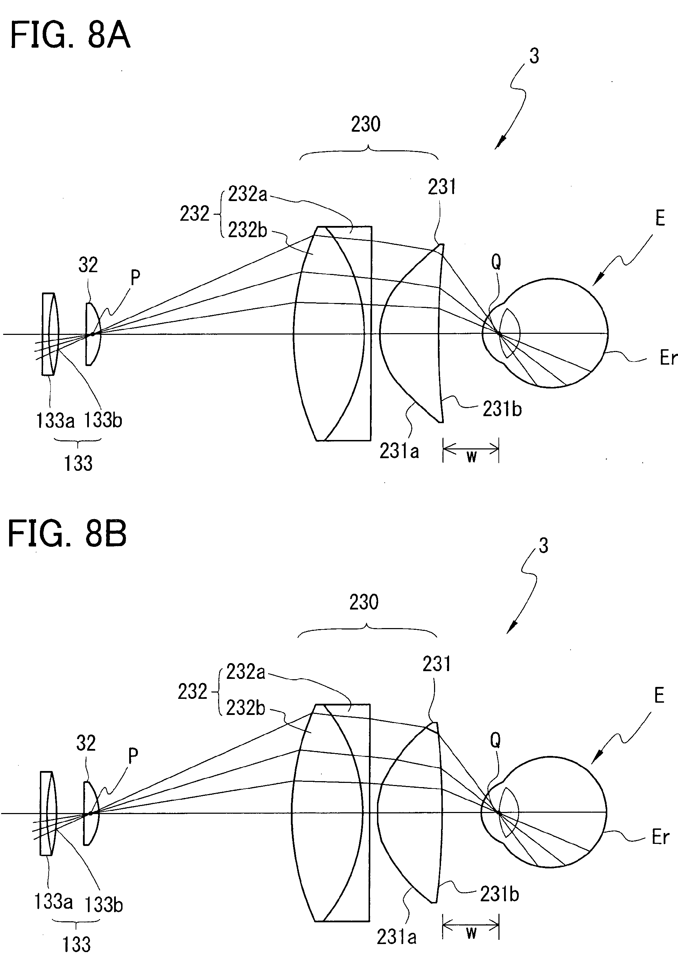

Although the above explanation shows the case where the examinee's-eye-side lens surface 231b is formed to be planar as one example, but it is not limited thereto. The examinee's-eye-side lens surface 231b may also be formed with a curved surface having a sufficiently large curvature radius. In this case, the examinee's-eye-side lens surface 231b may be either a concave surface or a convex surface. Even when the examinee's-eye-side lens surface 231 b is the concave surface or the convex surface, it is preferable to form the lens surface with a curvature radius ten times or more larger than a desired working distance. In this case, consequently, the second objective optical system 230 allowing the desired working distance to be obtained can be easily designed. For instance, as in examples in FIGs. 8A and 8B, in a case of the examinee's-eye-side lens surface 231b designed with a curvature radius of about 80 mm, it is easy to design the second objective optical system 230 for ensuring a working distance of about 8 mm (that is, w is settable to about 11 mm). Furthermore, the examinee's-eye-side lens surface 231b may be either spherical or aspherical. However, in a case of setting a photographable view angle larger, the light-source-side lens surface 231a of the first objective lens 231 has a shape more departing from a spherical surface. Therefore, as a method for producing the first objective lens 231, an aspherical surface forming method using cutting is considered to be easy to adopt. To reduce machining cost of a lens, accordingly, the examinee's-eye-side lens surface 231 b is preferably formed to be planar needing no cutting as shown in the aforementioned third embodiment.

The third embodiment uses, for the second objective lens 232, a cemented lens for correcting (reducing) magnification or lateral chromatic aberration occurring in the second objective optical system 230 due to at least two color laser beams different in wavelength. To be concrete, the second objective lens 232 reduces (diminishes) the lateral chromatic aberration caused by the second objective optical system 230 due to three color laser beams, red, green, and blue, emitted from the laser beam emitting part (light source) 11. Furthermore, the second objective lens 232 has positive power in total and bends the laser beams. As shown in FIG. 7, the second objective lens 232 bends the laser beams entering therein after traveling away from the optical axis L3 so as to come close to the optical axis L3 (for example, to travel towards the optical axis L3).

In this third embodiment, the second objective optical system 230 is a refracting optical system for bending the laser beams by refraction. This second optical system 230 in the third embodiment greatly bends the laser beams to ensure a view angle of 90° or more in full angle. In general, as a refracting component (e.g., a lens and a prism) bends a light beam more largely, larger lateral chromatic aberration may be caused. In the third embodiment, for successfully achieving the above-mentioned corresponding relationship, it is important to correct (reduce) the lateral chromatic aberration generated in the second objective optical system 230 due to the two or more colors laser beams (red, green, and blue in the third embodiment) emitted from the laser beam emitting part 11.

In the third embodiment, accordingly, the second objective lens 232 which is a cemented lens is provided in the second objective optical system 230, thereby reducing the lateral chromatic aberration generated in the second objective optical system 230 due to the three, red, green, and blue laser beams even while largely bending the laser beams to obtain a view angle of 90° or more in full angle. Consequently, the fundus photographing apparatus 1 in the third embodiment enables a good color fundus image to be produced.

Not all the lateral chromatic aberrations generated in the attached state of the wide-angle lens attachment 3 need to be corrected by the second objective lens 232. In a similar fashion to the second embodiment, the LCA correcting lens 133 may be used together to correct the lateral chromatic aberration. In this case, for example, a LCA correcting lens 133 providing 0 power (i.e., the sum of powers of the concave lens 133a and the convex lens 133b) is preferably used.

The second objective lens 232 is formed by bonding a concave lens 232a and a convex lens 232b. In the example of FIG. 7, a planar concave lens is used as the concave lens 232a and a double convex lens is used as the convex lens 232b. A concave surface of the concave lens 232a and one convex surface of the convex lens 232b are bonded to form the second objective lens 232. It is to be noted that the concrete shapes of the concave lens 232a and the convex lens 232b forming the second objective lens 232 are not limited to the above. In the third embodiment, the concave lens 232a and the convex lens 232b constituting the second objective lens 232 are different in light dispersion (Abbe's number) from each other. The thus configured second objective lens 232 reduces the lateral chromatic aberration generated when the light beams are largely bent by the second objective optical system 230.

In the second objective lens 232 in FIG. 7, the concave lens 232a is placed on a side close to the examinee's eye E with respect to the convex lens 232b. However, in the second objective lens 232, the positions of the concave lens 232a and the convex lens 232b may be reversed from those in the example of FIG. 7. That is, the concave lens 232a may be placed on a side close to the light source with respect to the convex lens 232b.

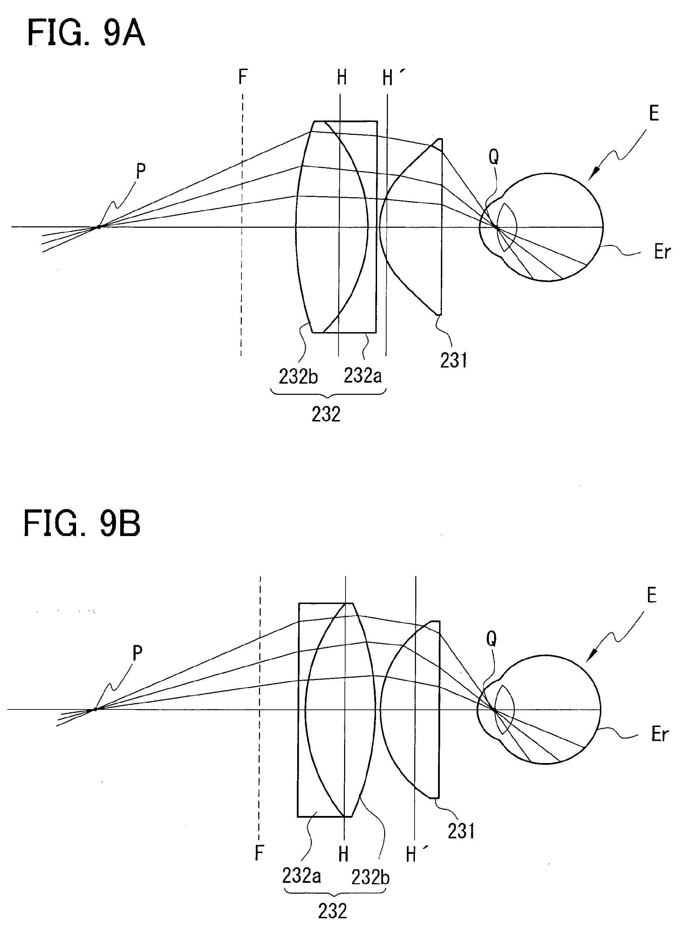

Herein, referring to FIGs. 9A and 9B, a comparison is made between a case where the concave lens 232a is placed on the examinee's eye E side with respect to the convex lens 232b (see FIG. 9A) and a case where the convex lens 232b is placed on the examinee's eye E side with respect to the concave lens 232a (see FIG. 9B). The optical system shown in FIG. 9A corresponds to a simplified illustration of the configuration of the wide-angle lens attachment) 3 shown in FIG. 7. It is further assumed that the optical system shown in FIG. 9B is designed with such a design solution as to obtain the distance w and the view angle equal to those in the optical system shown in FIG. 9A. Therefore, the lens designs assigned with the same reference signs in FIGs. 9A and 9B are not necessarily coincident with each other. In FIGs. 9A and 9B, H denotes the position of a front side principal point in the second objective optical system 230, H' denotes the position of a rear side principal point, and F denotes a focal point.

As shown in FIGs. 9A and 9B, when the orientation of the second objective lens 232 is reversed, the positions of the principal points H and H' are changed. In the example of FIG. 9A, in a case where the concave lens 232a is placed on the examinee's eye E side with respect to the convex lens 232b, the positions of the principal points H and H' can be located totally away from the second pivot point Q as compared with a case where the concave lens 232a is placed on the light source side with respect to the convex lens 232b. This results in that the example of FIG. 9B can reduce a paraxial imaging magnification from the first pivot point P to the second pivot point Q as compared with the example of FIG. 9A. For example, in one of the design solutions by the inventor, a value of the imaging magnification obtained in the example of FIG. 9A is about 0.414 (times) and a value of the imaging magnification obtained in the example of FIG. 9B is about 0.374 (times).

Even when the distances w and the view angles are equal as above, the imaging magnifications are different according to the design solutions of the second objective optical system 230. This is conceivably because the difference in imaging magnification depends on a satisfaction degree of a sine condition (a condition that further satisfies comma aberration in addition to satisfaction of spherical aberration) different between the example of FIG. 9A and the example of FIG. 9B. As the value of the imaging magnification is larger, the light density of a laser beam at a cornea is lower, so that a burden on an examinee's eye by the laser beam is reduced. Herein, the light density is proportional to the square of the imaging magnification. Accordingly, for example, when the imaging magnification in the example of FIG. 9A is about 0.414 (times) and the imaging magnification in the example of FIG. 9B is about 0.374 (times), the light density in the example of FIG. 9B is about 1.2 times that in the example of FIG. 9A. Thus, the example of FIG. 9A provides a lower light density of a laser beam at a cornea than in the example of FIG. 9B. The example of FIG. 9A is conceived as being able to relatively photograph the examinee's eye with reduced burden thereon.

When the orientation of the second objective lens 232 is reversed as shown in FIGs. 9A and 9B, it is found that the position of a focal point F in the second objective lens 230, that is, the fundus conjugate position of the examinee's eye E is changed. In more detail, the focal point F is located further away from the lens surface of each lens constituting the second objective optical system 230 in FIG. 9A than in FIG. 9B. In the fundus photographing apparatus 1, meanwhile, the light reflected by the lens surface of each lens on the light projecting optical path is removed by a confocal diaphragm (the pinhole plate 23 in the third embodiment, see FIG. 1). In this case, as the fundus conjugate position is more away from the lens surface (e.g., the light-source-side lens surface of the second objective lens 232) which reflects the laser beam toward the light source side, the light reflected by the lens surface is efficiently removed by the confocal diaphragm. Accordingly, the example of FIG. 9A can obtain a good fundus image with less noise.

On the other hand, as the value of the above-mentioned imaging magnification is smaller, the laser beam to be irradiated from the second objective optical system 230 to the examinee's eye E converges in a narrower range at the second pivot point Q. Thus, it is conceived that the laser beam to be irradiated to the examinee's eye E in the example of FIG. 9B is less likely to be vignetted by a pupil as compared with the example of FIG. 9A. Accordingly, the example of FIG. 9B is conceived to be relatively suitable for photographing an eye with a small pupil diameter, and others.

In the third embodiment, as described above, the first objective lens 231 which is an aspherical lens and the second objective lens 232 which is a cemented lens are arranged in combination to readily constitute the second objective optical system 230, thereby enabling photographing a good fundus image. For example, a fundus image with a wide view angle of 90° or more in full angle can be obtained successfully in a non-mydriatic state. Further, a good fundus image can be obtained with reduced influence of lateral chromatic aberration even in photographing using multi-color lights such as color photographing. Also, the working distance can be ensured well.

The above explanation is given to the embodiments; however, the above embodiments may be variously modified or changed.

For instance, each of the above embodiments shows the case where the image processing of vertically and horizontally reversing a fundus image according to the attached state of the wide-angle lens attachment 3 detected by the sensor 8, but it is not limited thereto. For instance, it may be configured to switch based on an instruction from an examiner between a first control mode of displaying a live image using an image having been subjected to the image processing of vertically and horizontally a fundus image and a second control mode of displaying an image using an image having been not subjected to the image processing. For instance, it may be arranged such that, upon receipt of a mode switching signal output based on a mode switching operation on an operation part not shown, the control unit 100 displays a live image in the control mode according to the mode switching signal.

Each of the above-described embodiments shows the case where the second objective optical systems 31 and 230 are provided in the wide-angle lens attachment 3 attachable to the apparatus main unit 2 remaining provided with the first objective optical system 16, but it is not limited thereto. For instance, the fundus photographing apparatus 1 may be an apparatus configured such that a lens attachment including the second objective optical systems 31 and 230 is attached to the apparatus main unit by replacement for a lens unit including the first objective optical system 16. In this case, the second objective optical systems 31 and 230 can also photograph a fundus image successfully with a wide view angle.

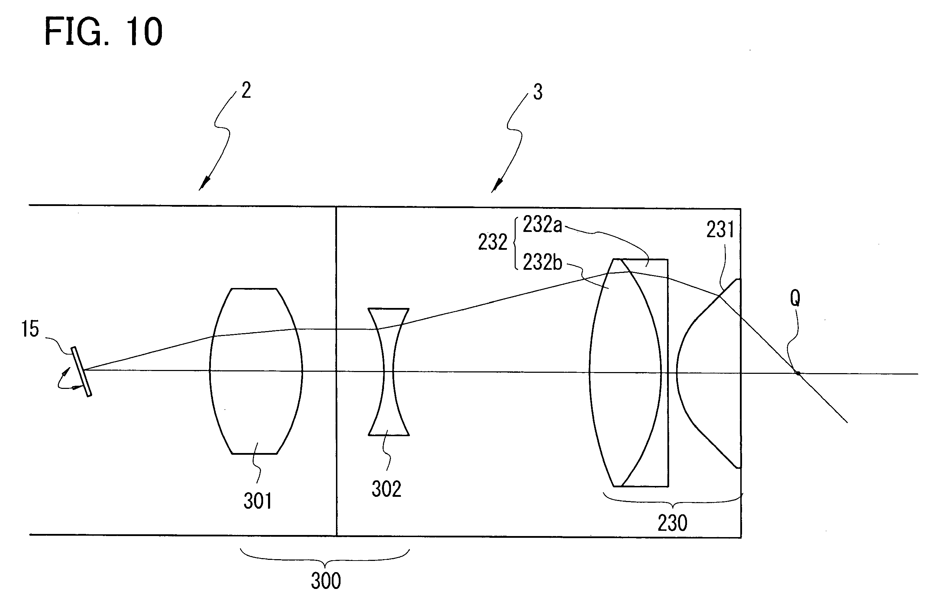

In this case, as exemplified in FIG. 10, a relay optical system such as a relay lens may be provided between the scanning part 15 and the second objective optical system (the second objective optical system 230 in FIG. 10). In FIG. 10, the optical system placed between the second objective optical system 230 and the scanning part 15 is one example of a relay optical system 300. In FIG. 10, the relay optical system 300 includes a plurality of lenses (lenses 301 and 302) and serves to deliver a laser beam reflected by the scanning part 15 to the second objective optical system 230. The lens shapes and positions in FIG. 10 are merely shown for convenience of explanation.

Furthermore, the third embodiment shows that an aspherical lens (the first objective lens 231) is placed as a lens on a closest side to an examinee's eye in the second objective optical system 230 and has an examinee's-eye-side lens surface (the lens surface 231b) formed with a curvature radius ten times or more larger than the working distance. However, a lens surface located on a closest side to an examinee's eye in the objective optical system (e.g., a second objective optical system) provided in the wide-angle lens attachment has only to be formed as a surface with a curvature radius ten times or more larger than the working distance. The lens formed with such a lens surface is not necessarily limited to an aspherical lens. For instance, it may be a spherical lens (e.g., the first objective lens 31a and others in the first and second embodiments) or a cemented lens. Also in those cases, it is conceivable that there is a case where such a design allowing easy ensuring of the working distance is facilitated.

The wide-angle lens attachment 3 in the first and second embodiments may include a cemented lens as in the third embodiment to reduce the lateral chromatic aberration generated in the second objective optical system 31. For instance, in the first and second embodiments, as shown in FIGs. 3 and 6, as one example, the wide-angle lens attachment 3 is provided with the third objective lens 31c which is a cemented lens. When this cemented lens consists of a concave lens and a convex lens appropriately made of materials different in light dispersion (Abbe's number), the lateral chromatic aberration generated by the second objective optical system 31 can be reduced by the third objective lens 31c. Of course, the configuration of the cemented lens is not limited to the configuration shown in FIGs. 3 and 6 and maybe changed appropriately.

The above embodiments explain the case where the photographing view angle of the scanning laser ophthalmoscope is widened by the wide-angle lens attachment, but it is not limited thereto. For instance, the technique disclosed in the embodiments is also applicable to an Optical Coherence Tomography (OCT) which is one type of the fundus photographing apparatus. The OCT has an interference optical system for splitting light emitted from a light source into a laser beam and a reference light beam, delivering the laser beam to a predetermined portion of an examinee's eye and delivering the reference light beam to a reference optical system, and then causing a light receiving element to receive interference light obtained by synthesizing the laser beam reflected by the predetermined portion of the examinee's eye and the reference light beam. The OCT further has a scanning part for scanning the laser beam on a fundus.

It is to be noted that the fundus photographing apparatus 1 disclosed in the above embodiments can also be defined as the following apparatus.

Specifically, a first fundus photographing apparatus includes: a light source configured to emit at least a laser beam having a first wavelength and a laser beam having a second wavelength different from the first wavelength; a scanning part configured to change a traveling direction of the laser beam emitted from the light source to scan the laser beam on a fundus; and an objective optical system configured to form a pivot point about which the laser beam delivered from the scanning part is caused to pivot in association with operation of the scanning part, the objective optical system being placed between an examinee's eye and the scanning part, the fundus photographing apparatus being configured to image a fundus image of the examinee's eye by use of the light from the fundus resulting from the laser beam having passed the pivot point and been delivered to the fundus of the examinee's eye, wherein the objective optical system bends the laser beam delivered therein from the scanning part toward an optical axis of the objective optical system to provide a scanning range of the laser beam on the fundus with a full angle of 90° or more, and the objective optical system further includes at least a cemented lens for correcting lateral chromatic aberration caused in the objective optical system due to at least the laser beam of the first wavelength and the laser beam of the second wavelength.

In a second fundus photographing apparatus according to the first fundus photographing apparatus, the cemented lens includes a single concave lens and a single convex lens that are bonded to each other, the lenses being made of materials different in dispersion.

In a third fundus photographing apparatus according to the second fundus photographing apparatus, the cemented lens is configured such that the concave lens is placed on a side close to the examinee's eye with respect to the convex lens.

In a fourth fundus photographing apparatus according to the second fundus photographing apparatus, the cemented lens is configured such that the convex lens is placed on a side close to the examinee's eye with respect to the concave lens.

In the fifth fundus photographing apparatus according to any one of the first to fourth fundus photographing apparatuses, the objective optical system includes, as a lens placed on a closest side to the examinee's eye in the objective optical system, an aspherical lens having at least one lens surface formed to be aspheric for reducing spherical aberration of imaging at the pivot point.

In the sixth fundus photographing apparatus according to the fifth fundus photographing apparatuses, the one lens surface formed aspheric in the aspherical lens is a curved surface formed to have a curvature radius larger as being away from the optical axis of the aspherical lens.

In a seventh fundus photographing apparatus according to the fifth or sixth fundus photographing apparatuses, the objective optical system includes only the aspherical lens and the cemented lens, and these aspherical lens and cemented lens bend a laser beam from the scanning part toward the optical axis of the objective optical system from a highest position of ray height.

In an eighth fundus photographing apparatus according to any one of the first to seventh fundus photographing apparatuses, an examinee's-eye-side lens surface formed on a closest side to the examinee's eye in the objective optical system is a surface having a curvature radius ten times or more larger than a working distance.

In a ninth fundus photographing apparatus according to the eighth fundus photographing apparatus, the examinee's-eye-side lens surface in the aspherical lens is planar.

In a tenth fundus photographing apparatus according to any one of the first to ninth fundus photographing apparatuses, there is provided a wide-angle lens attachment attachable to a housing surface of the fundus photographing apparatus on a side which will face to an examinee, and the objective optical system is provided in the wide-angle lens attachment.

Furthermore, the wide-angle lens attachment 3 disclosed in the above embodiments can also be defined as the following apparatus.

Specifically, the wide-angle lens attachment includes: a light source configured to emit at least a laser beam having a first wavelength and a laser beam having a second wavelength different from the first wavelength; a scanning part configured to change a traveling direction of the laser beam emitted from the light source to scan the laser beam on a fundus of an examinee's eye; and a first objective optical system being placed between an examinee's eye and the scanning part and configured to form a first pivot point about which the laser beam delivered from the scanning part is caused to pivot in association with operation of the scanning part, the wide-angle lens attachment being attachable to a housing surface of the fundus photographing apparatus on a side which will face to an examinee, the fundus photographing apparatus being configured to image an image of the fundus by use of light from the fundus resulting from the laser beam having passed the first pivot point and being delivered to the fundus of the examinee's eye, the attachment being configured to widen an imaging view angle of the image of the fundus, wherein in an attached state where the attachment is attached to the housing surface having an inspection window, a second objective optical system is provided to form a second pivot point about which the laser beam having passed the first pivot point is further caused to pivot in association with the operation of the scanning part, and the second objective optical system includes at least a cemented lens for correcting at least lateral chromatic aberration caused in the first objective optical system due to the laser beam of the first wavelength and the laser beam of the second wavelength.

**Claims**

1. A fundus photographing apparatus (1) including: a scanning part (15) configured to change a traveling direction of a laser beam emitted from a light source (11) to scan the laser beam on a fundus (Er); and a first objective optical system (16) placed between an examinee's eye (E) and the scanning part to form a first pivot point (P) about which the laser beam delivered from the scanning part is caused to pivot in association with operation of the scanning part, the fundus photographing apparatus being configured to image an image of the fundus of the examinee's eye by use of light from the fundus resulting from the laser beam having passed the first pivot point and being delivered to the fundus of the examinee's eye, wherein

   the apparatus further includes a wide-angle lens attachment (3) attachable to a housing surface of the apparatus on a side which will face to the examinee, the housing surface having an inspection window (17), and the wide-angle lens attachment (3) being configured to widen an imaging view angle of the image of the fundus,

   the wide-angle lens attachment is provided with a second objective optical system (31; 230) for forming a second pivot point (Q) about which the laser beam having passed the first pivot point is further caused to pivot in association with the operation of the scanning part in an attached state where the attachment is attached to the housing surface, and

   the second objective optical system includes at least a lens (31a, 31b, 31c; 231, 232) placed to bend the laser beam toward an optical axis (L3) of the second objective optical system at a higher position than a chief ray height (h1) of the laser beam on the inspection window in the attached state.

2. The fundus photographing apparatus according to claim 1 further including reversal processing means (100) for creating an image of the fundus by vertically and horizontally reversing pixel arrangement corresponding to a scanning order of the scanning part from an image (I) of the fundus imaged in a non-attached state of the wide-angle lens attachment.

3. The fundus photographing apparatus according to claim 1 or 2, wherein the wide-angle lens attachment includes a diopter correcting lens optical system (32) for canceling out diopter change caused by the second objective optical system.

4. The fundus photographing apparatus according to claim 3, wherein the diopter correcting lens optical system is positioned at the first pivot point.

5. The fundus photographing apparatus according to any one of claims 1 to 4, wherein the wide-angle lens attachment includes a lateral chromatic aberration correcting lens optical system (133) for reducing lateral chromatic aberration caused by at least the second objective optical system.

6. The fundus photographing apparatus according to claim 5, wherein the lateral chromatic aberration correcting lens optical system is placed between the first objective optical system (16) and the first pivot point (P) in the attached state, and includes a lens surface on a side closest to the inspection window (17) to form an entrance window for a laser beam in the wide-angle lens attachment.

7. The fundus photographing apparatus according to any one of claims 1 to 6, wherein the second objective optical system (230) includes an aspherical lens (231) for correcting spherical aberration of imaging at the second pivot point.

8. The fundus photographing apparatus according to claim 7, wherein the aspherical lens (231) is a lens placed on a side closest to the examinee's eye in the second objective optical system, and has an examinee's-eye-side lens surface (231b) formed as a surface having a curvature radius ten times or more larger than a working distance and a light-source-side lens surface (231a) formed as an aspherical surface.

9. The fundus photographing apparatus according to claim 8, wherein the examinee's-eye-side lens surface of the aspherical lens is planar.

**10.** The fundus photographing apparatus according to any one of claims 7 to 9, wherein the light-source-side lens surface of the aspherical lens is a curved surface having a curvature radius increasing in a position farther from the optical axis of the aspherical lens.

**11.** The fundus photographing apparatus according to any one of claims 1 to 10, wherein
the light source (11) is configured to emit a laser beam having a first wavelength and a laser beam having a second wavelength different from the first wavelength, and
the second objective optical system (230) includes a cemented lens (232) for correcting lateral chromatic aberration generated in the second objective optical system due to the laser beam of the first wavelength and the laser beam of the second wavelength.

**12.** The fundus photographing apparatus according to any one of claims 1 to 6, wherein
the light source is configured to emit a laser beam having a first wavelength and a laser beam having a second wavelength different from the first wavelength, and
the second objective optical system includes only:

an aspherical lens (231) for correcting spherical aberration of imaging at the second pivot point; and
a cemented lens (232) placed on a side close to the light source with respect to the aspherical lens and configured to correct lateral chromatic aberration generated in the second objective optical system due to the laser beam of the first wavelength and the laser beam of the second wavelength.

**13.** A wide-angle lens attachment (3) including: a scanning part (15) for changing a traveling direction of a laser beam emitted from a light source (11) to scan the laser beam on a fundus (Er) of an examinee's eye (E); and a first objective optical system (16) placed between the examinee's eye (E) and the scanning part and configured to form a first pivot point (P) about which the laser beam delivered from the scanning part is caused to pivot in association with operation of the scanning part, the wide-angle lens attachment (3) being attachable to a housing surface of a fundus photographing apparatus (1) on a side which will face an examinee, the fundus photographing apparatus being configured to image an image of the fundus of the examinee's eye by use of light from the fundus resulting from the laser beam having passed the first pivot point and being delivered to the fundus of the examinee's eye, the attachment being configured to widen an imaging view angle of the image of the fundus,

wherein
a second objective optical system (31; 230) is provided to form a second pivot point (Q) about which the laser beam having passed the first pivot point is further caused to pivot in association with the operation of the scanning part in an attached state where the attachment is attached to the housing surface having an inspection window (17), and
the second objective optical system includes at least a lens (31a, 31b, 31c; 231, 232) placed to bend the laser beam toward an optical axis of the second objective optical system at a higher position than a chief ray height (h1) of the laser beam on the inspection window in the attached state.

**Patentansprüche**

**1.** Augenhintergrundfotografiergerät (11) mit: einem Abtastteil (15), der dazu konfiguriert ist, eine Bewegungsrichtung eines Laserstrahls zu ändern, der von einer Lichtquelle (11) ausgesendet wird, um den Laserstrahl an einem Augenhintergrund (Er) abzutasten; und einem ersten optischen Objektivsystem (16), das zwischen einem Auge (E) eines Prüflings und dem Abtastteil platziert ist, um einen ersten Wendepunkt (P) zu bilden, um den der Laserstrahl, der von dem Abtastteil geliefert wird, im Zusammenhang mit einem Betrieb des Abtastteils zum Wenden veranlasst wird, wobei das Augenhintergrundfotografiergerät dazu konfiguriert ist, ein Bild des Augenhintergrunds des Auges des Prüflings unter Verwendung von Licht von dem Augenhintergrund abzubilden, das aus dem Laserstrahl resultiert, der den ersten Wendepunkt passiert hat und zu dem Augenhintergrund des Auges des Prüflings geliefert wird,
wobei das Gerät des Weiteren eine Weitwinkellinsenanbringung (3) aufweist, die an einer Gehäusefläche des Gerätes an einer Seite anbringbar ist, die dem Prüfling zuzuwenden ist, wobei die Gehäusefläche ein Prüffenster (17) hat, und die Weitwinkellinsenanbringung (3) dazu konfiguriert ist, einen Abbildungsbildwinkel des Bildes des Augenhintergrundes aufzuweiten,
wobei die Weitwinkellinsenanbringung mit einem zweiten optischen Objektsystem (31; 230) zum Bilden eines zweiten Wendepunktes (Q) versehen ist, um den der Laserstrahl, der den ersten Wendepunkt passiert hat, des Weiteren im Zusammenhang mit dem Betrieb des Abtastteils in einem angebrachten Zustand, in dem die Anbringung an der Gehäusefläche angebracht ist, zum Wenden veranlasst wird, und
wobei das zweite optische Objektivsystem zumindest eine Linse (31a, 31b, 31c; 231, 232) aufweist, die zum Beugen des Laserstrahls zu einer optischen Achse (L3) des zweiten optischen Objektivsystems an einer Position platziert ist, die höher ist als eine Hauptstrahlhöhe (H1) des Laserstrahls in dem Prüffenster in dem angebrachten Zustand.

2. Augenhintergrundfotografiergerät gemäß Anspruch 1, des Weiteren mit einer Umkehrverarbeitungseinrichtung (100) zum Erzeugen eines Bildes des Augenhintergrundes durch vertikales und horizontales Umkehren einer Pixelanordnung entsprechend einer Abtastreihenfolge des Abtastteils von einem Bild (I) des Augenhintergrundes, das in einem nicht-angebrachten Zustand der Weitwinkellinsenanbringung abgebildet ist.

3. Augenhintergrundfotografiergerät gemäß Anspruch 1 oder 2, wobei die Weitwinkellinsenanbringung ein optisches Dioptrienkorrekturlinsensystem (32) aufweist, um eine Dioptrienänderung zu beseitigen, die durch das zweite optische Objektivsystem verursacht wird.

4. Augenhintergrundfotografiergerät gemäß Anspruch 3, wobei das optische Dioptrienkorrekturlinsensystem an dem ersten Wendepunkt positioniert ist.

5. Augenhintergrundfotografiergerät gemäß einem der Ansprüche 1 bis 4, wobei die Weitwinkellinsenanbringung ein optisches, laterales, chromatisches Aberrations-Korrekturlinsensystem (133) zum Reduzieren einer lateralen, chromatischen Aberration aufweist, die zumindest durch das zweite optische Objektivsystem verursacht wird.

6. Augenhintergrundfotografiergerät gemäß Anspruch 5, wobei das optische, laterale, chromatische Aberrations-Korrekturlinsensystem zwischen dem ersten optischen Objektivsystem (16) und dem ersten Wendepunkt (P) in dem angebrachten Zustand platziert ist und eine Linsenfläche an jener Seite aufweist, die dem Prüffenster (17) am nächsten ist, um ein Eingangsfenster für einen Laserstrahl in die Weitwinkellinsenanbringung zu bilden.

7. Augenhintergrundfotografiergerät gemäß einem der Ansprüche 1 bis 6, wobei das zweite optische Objektivsystem (230) eine asphärische Linse (231) aufweist, um eine sphärische Aberration der Abbildung an dem zweiten Wendepunkt zu korrigieren.

8. Augenhintergrundfotografiergerät gemäß Anspruch 7, wobei die asphärische Linse (231) eine Linse ist, die an jener Seite platziert ist, die dem Auge des Prüflings in dem zweiten optischen Objektivsystem am nächsten ist, und die eine Linsenfläche (231b) an der Seite des Auges des Prüflings, die als eine Fläche ausgebildet ist, welche einen Krümmungsradius hat, der das Zehnfache oder mehr größer als ein Arbeitsabstand ist, und eine Linsenfläche (231a) an der Seite der Lichtquelle hat, die als eine asphärische Fläche ausgebildet ist.

9. Augenhintergrundfotografiergerät gemäß Anspruch 8, wobei die Linsenfläche an der Seite des Auges des Prüflings von der asphärischen Linse planar ist.

10. Augenhintergrundfotografiergerät gemäß einem der Ansprüche 7 bis 9, wobei die Linsenfläche an der Seite der Lichtquelle von der asphärischen Linse eine gekrümmte Fläche ist, die einen Krümmungsradius hat, der sich an einer Position vergrößert, die von der optischen Achse der asphärischen Linse weiter weg ist.

11. Augenhintergrundfotografiergerät gemäß einem der Ansprüche 1 bis 10, wobei
die Lichtquelle (11) dazu konfiguriert ist, einen Laserstrahl mit einer ersten Wellenlänge und einen Laserstrahl mit einer zweiten Wellenlänge auszusenden, die sich von der ersten Wellenlänge unterscheidet, und
das zweite optische Objektivsystem (230) eine verkittete Linse (232) aufweist, um eine laterale, chromatische Aberration zu korrigieren, die in dem zweiten optischen Objektivsystem aufgrund des Laserstrahls der ersten Wellenlänge und des Laserstrahls der zweiten Wellenlänge erzeugt wird.

12. Augenhintergrundfotografiergerät gemäß einem der Ansprüche 1 bis 6, wobei
die Lichtquelle dazu konfiguriert ist, einen Laserstrahl mit einer ersten Wellenlänge und einen Laserstrahl mit einer zweiten Wellenlänge auszusenden, die sich von der ersten Wellenlänge unterscheidet, und
das zweite optische Objektivsystem nur Folgendes aufweist:

eine asphärische Linse (231) zum Korrigieren einer sphärischen Aberration einer Abbildung an dem zweiten Wendepunkt; und
eine verkittete Linse (232), die an einer Seite nahe der Lichtquelle hinsichtlich der asphärischen Linse platziert und dazu konfiguriert ist, eine laterale, chromatische Aberration zu korrigieren, die in dem zweiten optischen Objektivsystem aufgrund des Laserstrahls der ersten Wellenlänge und des Laserstrahls der zweiten Wellenlänge erzeugt wird.

13. Weitwinkellinsenanbringung (3) mit:

einem Abtastteil (15) zum Ändern einer Bewegungsrichtung eines Laserstrahls, der von einer Lichtquelle (11) ausgesendet wird, um den Laserstrahl an einem Augenhintergrund (Er) eines Auges (E) eines Prüflings abzutasten; und einem ersten optischen Objektivsystem (16), das zwischen dem Auge (E) des Prüflings und dem Abstastteil platziert und dazu konfiguriert ist, einen ersten Wendepunkt (P) zu bilden, um den der Laserstrahl, der von dem Abstastteil geliefert wird, im Zusammenhang mit einem Betrieb des Abstastteils zum Wenden veranlasst wird, wobei die Weitwinkellinsenanbringung (3) an einer Gehäusefläche eines Augenhintergrundfotografiergerätes (1) an einer Seite anbringbar ist, die einem Prüfling zuzuwenden ist, wobei das Augenhintergrundfotografiergerät dazu konfiguriert ist, ein Bild des Augenhintergrunds des Auges des Prüflings durch Nutzung von Licht von dem Augenhintergrund abzubilden, das von dem Laserstrahl resultiert, der den ersten Wendepunkt passiert hat und zu dem Augenhintergrund des Auges des Prüflings geliefert wird, wobei die Anbringung dazu konfiguriert ist, einen Abbildungsbildwinkel des Bildes des Augenhintergrunds aufzuweiten,
wobei ein zweites optisches Objektivsystem (31; 230) vorgesehen ist, um einen zweiten Wendepunkt (Q) zu bilden, um den der Laserstrahl, der den ersten Wendepunkt passiert hat, des Weiteren im Zusammenhang mit dem Betrieb des Abstastteils in einem angebrachten Zustand, in dem die Anbringung an der Gehäusefläche angebracht ist, die ein Prüffenster (17) hat, zum Wenden veranlasst wird, und
wobei das zweite optische Objektivsystem zumindest eine Linse (31a, 31b, 31c; 231, 232) aufweist, die zum Beugen des Laserstrahls zu einer optischen Achse des zweiten optischen Objektivsystems an einer Position platziert ist, die höher ist als eine Hauptstrahlhöhe (H1) des Laserstrahls in dem Prüffenster in dem angebrachten Zustand.

## Revendications

1. Dispositif de photographie de fond d'oeil (1) comportant : une partie de balayage (15) configurée de manière à modifier une direction de déplacement d'un faisceau laser émis à partir d'une source de lumière (11) afin de balayer le faisceau laser sur un fond d'oeil (Er) ; et un premier dispositif optique formant objectif (16) placé entre un oeil de patient (E) et la partie de balayage afin de former un premier point de pivot (P) autour duquel le faisceau laser délivré à partir de la partie de balayage est amené à pivoter en association avec le fonctionnement de la partie de balayage, le dispositif de photographie de fond d'oeil étant configuré de manière à former une image du fond d'oeil de l'oeil du patient en utilisant la lumière à partir du fond d'oeil résultant du passage du faisceau laser par le premier point de pivot et qui est délivrée au fond d'oeil de l'oeil du patient,
dans lequel
le dispositif comporte en outre un adaptateur formant objectif grand angle (3) pouvant être fixé sur une surface de boîtier du dispositif sur un côté qui fait face au patient, la surface de boîtier présentant une fenêtre d'inspection (17), et l'adaptateur formant objectif grand angle (3) étant configuré de manière à agrandir un angle de vue de formation d'image de l'image du fond d'oeil,
l'adaptateur formant objectif grand angle comporte un second dispositif optique formant objectif (31 ; 230) destiné à former un second point de pivot (Q) autour duquel le faisceau laser qui est passé par le premier point de pivot est en outre amené à pivoter en association avec le fonctionnement de la partie de balayage dans un état fixé dans lequel l'adaptateur est fixé sur la surface de boîtier, et
le second dispositif optique formant objectif comporte au moins une lentille (31a, 31b, 31c ; 231, 232) placée de manière à orienter le faisceau laser vers un axe optique (L3) du second dispositif optique formant objectif à une position supérieure à une hauteur de rayon maximum (h1) du faisceau laser sur la fenêtre d'inspection dans l'état fixé.

2. Dispositif de photographie de fond d'oeil selon la revendication 1, comportant en outre un moyen de traitement d'inversion (100) destiné à créer une image du fond d'oeil par inversion verticale et horizontale de l'agencement de pixel correspondant à un ordre de balayage de la partie de balayage à partir d'une image (I) du fond d'oeil prise dans un état non fixé de l'adaptateur formant objectif grand angle.

3. Dispositif de photographie de fond d'oeil selon la revendication 1 ou 2, dans lequel l'adaptateur formant objectif grand angle comporte un dispositif optique formant lentille de correction de dioptre (32) destiné à annuler une modification de dioptre provoquée par le second dispositif optique formant objectif.

4. Dispositif de photographie de fond d'oeil selon la revendication 3, dans lequel le dispositif optique formant lentille de correction de dioptre est positionné au niveau du premier point de pivot.

5. Dispositif de photographie de fond d'oeil selon l'une quelconque des revendications 1 à 4, dans lequel l'adaptateur formant objectif grand angle comporte un dispositif optique formant lentille de correction d'aberration chromatique latérale (133) destiné à réduire l'aberration chromatique latérale provoquée au moins par le second dispositif optique formant objectif.

6. Dispositif de photographie de fond d'oeil selon la revendication 5, dans lequel le dispositif optique formant lentille de correction d'aberration chromatique latérale est placé entre le premier dispositif optique formant objectif (16) et le premier point de pivot (P) dans l'état fixé, et comporte une surface de lentille sur un côté le plus proche de la fenêtre d'inspection (17) afin de former une fenêtre d'entrée pour un faisceau laser sur l'adaptateur formant objectif grand angle.

7. Dispositif de photographie de fond d'oeil selon l'une quelconque des revendications 1 à 6, dans lequel le second dispositif optique formant objectif (230) comporte une lentille asphérique (231) destinée à corriger l'aberration sphérique de formation d'image au niveau du second point de pivot.

8. Dispositif de photographie de fond d'oeil selon la revendication 7, dans lequel la lentille asphérique (231) est une lentille placée sur le côté le plus proche de l'oeil de patient dans le second dispositif optique formant objectif, et présente une surface de lentille du côté oeil de patient (231b) formée comme une surface présentant un rayon de courbure dix fois supérieur ou plus à une distance active et une surface de lentille du côté source de lumière (231a) formée comme une surface asphérique.

9. Dispositif de photographie de fond d'oeil selon la revendication 8, dans lequel la surface de lentille du côté oeil de patient de la lentille asphérique est plane.

10. Dispositif de photographie de fond d'oeil selon l'une quelconque des revendications 7 à 9, dans lequel la surface de lentille du côté de source de lumière de la lentille asphérique est une surface courbe présentant un rayon de courbure croissant à une position s'éloignant de l'axe optique de la lentille asphérique.

11. Dispositif de photographie de fond d'oeil selon l'une quelconque des revendications 1 à 10, dans lequel la source de lumière (11) est configurée de manière à émettre un faisceau laser présentant une première longueur d'onde et un faisceau laser présentant une seconde longueur d'onde différente de la première longueur d'onde, et le second dispositif optique formant objectif (230) comporte une lentille cimentée (232) destinée à corriger une aberration chromatique latérale produite dans le second dispositif optique formant objectif du fait du faisceau laser de la première longueur d'onde et du faisceau laser de la seconde longueur d'onde.

12. Dispositif de photographie de fond d'oeil selon l'une quelconque des revendications 1 à 6, dans lequel la source de lumière est configurée de manière à émettre un faisceau laser présentant une première longueur d'onde et un faisceau laser présentant une seconde longueur d'onde différente de la première longueur d'onde, et le second dispositif optique formant objectif comporte uniquement :

une lentille asphérique (231) destinée à corriger une aberration sphérique de formation d'image au niveau du second point de pivot ; et une lentille cimentée (232) placée sur un côté proche de la source de lumière par rapport à la lentille asphérique et configurée de manière à corriger une aberration chromatique latérale produite dans le second dispositif optique formant objectif du fait du faisceau laser de la première longueur d'onde et du faisceau laser de la seconde longueur d'onde.

13. Adaptateur formant objectif grand angle (3) comportant : une partie de balayage (15) destinée à modifier une direction de déplacement d'un faisceau laser émis à partir d'une source de lumière (11) afin de balayer le faisceau laser sur un fond d'oeil (Er) d'un oeil de patient (E) ; et un premier dispositif optique formant objectif (16) placé entre l'oeil de patient (E) et la partie de balayage et configuré de manière à former un premier point de pivot (P) autour duquel le faisceau laser délivré à partir de la partie de balayage est amené à pivoter en association avec le fonctionnement de la partie de balayage, l'adaptateur formant objectif grand angle (3) pouvant être fixé sur une surface de boîtier d'un dispositif de photographie de fond d'oeil (1) sur un côté qui fait face à un patient, le dispositif de photographie de fond d'oeil étant configuré de manière à prendre une image d'une image du fond d'oeil de l'oeil du patient en utilisant la lumière provenant du fond d'oeil résultant du passage du faisceau laser par le premier point de pivot et délivrée sur le fond d'oeil de l'oeil du patient, l'adaptateur étant configuré de manière à agrandir un angle de vue de formation d'image de l'image du fond d'oeil, dans lequel un second dispositif optique formant objectif (31 ; 230) est prévu afin de former un second point de pivot (Q) autour duquel le faisceau laser qui est passé par le premier point de pivot est en outre amené à pivoter en association avec le fonctionnement de la partie de balayage dans un état fixé dans lequel l'adaptateur est fixé sur la surface de boîtier comportant une fenêtre d'inspection (17), et le second dispositif optique formant objectif comporte au moins une lentille (31a, 31b, 31c ; 231, 232) placée de manière à orienter le faisceau laser vers un axe optique du second dispositif optique formant objectif à une position supérieure à une hauteur de rayon maximum (h1) du faisceau laser sur la fenêtre d'inspection dans l'état fixé.

# FIG. 1

# FIG. 2

EP 2 901 919 B1

# FIG. 3

# FIG. 4

FIG. 5A

<NON-ATTACHED STATE OF ATTACHMENT>

I

1
2
3
4

N

FIG. 5B

<ATTACHED STATE OF ATTACHMENT>

I

N

4
3
2
1

FIG. 6

EP 2 901 919 B1

FIG. 7

EP 2 901 919 B1

# FIG. 8A

# FIG. 8B

# FIG. 9A

# FIG. 9B

# FIG. 10

EP 2 901 919 B1

**EP 2 901 919 B1**

**REFERENCES CITED IN THE DESCRIPTION**

**Patent documents cited in the description**

- JP 2009011381 A